(19) Europäisches Patentamt
European Patent Office
Office européen des brevets

(11) Veröffentlichungsnummer: **0 476 649 A2**

(12) **EUROPÄISCHE PATENTANMELDUNG**

(21) Anmeldenummer: **91115935.8**

(51) Int. Cl.5: **C07D 477/00**

(22) Anmeldetag: **19.09.91**

(30) Priorität: **20.09.90 DE 4029731**
**18.10.90 DE 4033033**

(43) Veröffentlichungstag der Anmeldung:
**25.03.92 Patentblatt 92/13**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Budt, Karl-Heinz, Dr.**
**Am Flachsland 18**
**W-6233 Kelkheim/Taunus(DE)**
Erfinder: **Hörlein, Rolf, Dr.**
**Assmannshäuser Weg 21**
**W-6000 Frankfurt am Main(DE)**
Erfinder: **Lattrell, Rudolf, Dr.**
**Heuhohlweg 6h**
**W-6240 Königstein/Taunus(DE)**
Erfinder: **Wollmann, Theodor, Dr.**
**Gartenstrasse 25**
**W-6238 Hofheim am Taunus(DE)**

(54) **Verfahren zur Herstellung von Carbapenemverbindungen.**

(57)

Verbindung I

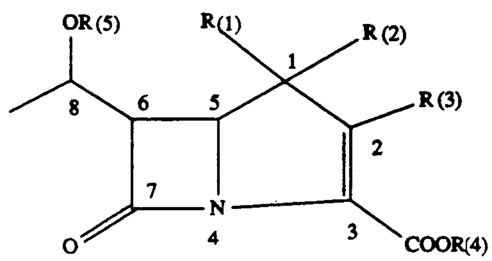

I

erhält man durch Umsetzung von Verbindung IV

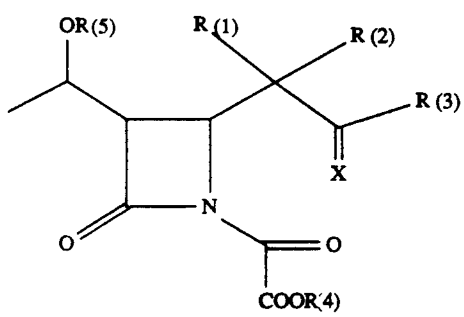

IV

mit Verbindung V

EP 0 476 649 A2

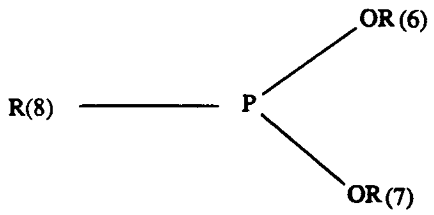

V

bei Temperaturen von 50 bis 180 ° C in einem geeigneten organischen Lösungsmittel.

Die Erfindung betrifft ein Verfahren zur Herstellung von Carbapenemverbindungen.

Carbapenemderivate der Formel I sind wertvolle Verbindungen mit antibiotischen Eigenschaften. Literaturbekannte Syntheseverfahren bedienen sich der Zwischenstufen der Verbindungen der Formeln II und III, deren Herstellung langwierig ist:

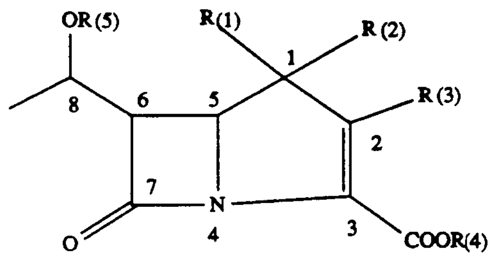
I

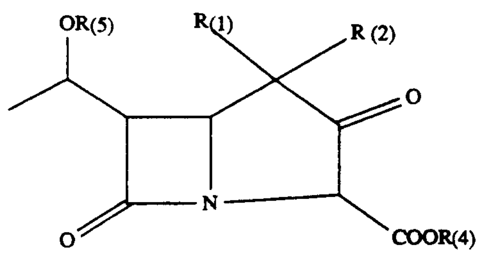
II

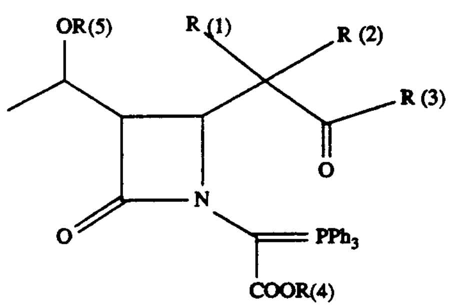
III

Eine weitere Verfahrensvariante besteht in der Cyclisierung der Vorstufe IV (Oxalimidcyclisierung):

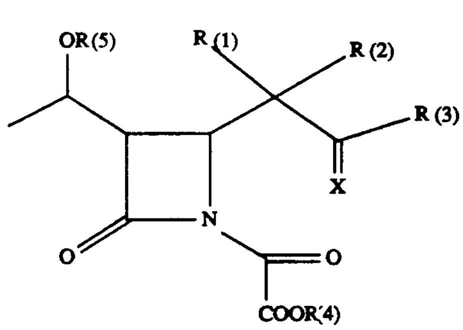
IV

In der Literatur wird als Cyclisierungsreagenz ausschließlich Trimethylphosphit oder Triethylphosphit verwendet. Nachteilig sind die oft sehr niedrigen Ausbeuten aufgrund der notwendigen hohen Reaktionstemperaturen und langen Reaktionszeiten.

Es wurde nun überraschenderweise gefunden, daß sich diese Nachteile vermeiden lassen, wenn man die Zyklisierung der Vorstufe IV unter Verwendung von Alkylphosphonigsäuredialkylestern ausführt. Diese neuartigen Reagenzien führen im Vergleich mit Alkylphosphiten zu einer Zyklisierung unter wesentlich milderen Reaktionsbedingungen und damit zu wesentlich höheren Ausbeuten an Endprodukt I.

Die Erfindung betrifft daher ein Verfahren zur Herstellung von Carbapenemderivaten der Formel I

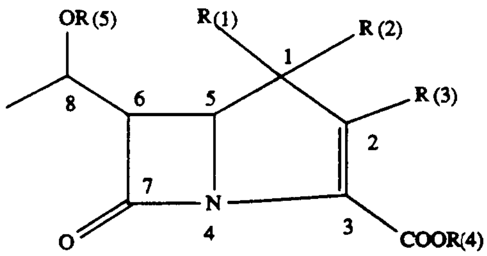

I

worin bedeuten:

R(1), R(2) Wasserstoff, ($C_1$-$C_4$)-Alkyl, ($C_1$-$C_4$)-Alkenyl, ($C_1$-$C_4$)-Alkoxy, ($C_4$-$C_7$)-Cycloalkyl oder ($C_3$-$C_6$)-Spirocyclyl,

R(3) Wasserstoff, ($C_1$-$C_4$)-Alkyl, ($C_1$-$C_{12}$)-Alkylthio [wobei die Alkylgruppen unsubstituiert sind oder ein- oder zweifach substituiert durch Hydroxy, geschütztes Hydroxy, ($C_1$-$C_4$)-Alkoxy, ($C_1$-$C_4$)-Alkyloxycarbonyl,($C_1$-$C_4$)-Acyloxy, Amino, ($C_1$-$C_4$)-Alkylamino, ($C_1$-$C_4$)-Acylamino, Thiol, ($C_1$-$C_4$)-Alkylthio oder Heterocyclylthio, z. B. Thiazolyl-, Thiadiazolyl-, Pyridylthio], Phenyl, Heterocyclyl, Phenylthio, Heterocyclylthio [wobei die Phenyl- und Heterocyclylringe unsubstituiert oder ein- oder zweifach substituiert sind durch Hydroxy, geschütztes Hydroxy, Carboxy, ($C_1$-$C_4$)-Alkoxycarbonyl, Allyloxycarbonyl, Aminocarbonyl, ($C_1$-$C_4$)-Alkylaminocarbonyl, Cyano, F, Cl, Br], ($C_3$-$C_6$)-Cycloalkyl, ($C_3$-$C_6$)-Cycloalkylthio, ($C_5$-$C_6$)-Oxacycloalkyl [gesättigt, ein- oder zweifach ungesättigt], ($C_3$-$C_6$)-Oxocycloalkyl, ($C_3$-$C_6$)-[1,1-bis-($C_1$-$C_3$)-Alkyloxy]-cycloalkyl, ($C_3$-$C_6$)-[($C_1$-$C_3$)-Alkylimino]-cycloalkyl, ($C_3$-$C_6$)-[Phenylimino]-cycloalkyl,($C_3$-$C_6$)-(Hydroxyimino)-cycloalkyl, ($C_3$-$C_6$)-[($C_1$-$C_3$)-Alkyloxyimino]-cycloalkyl, in denen der Cycloalkylrest unsubstituiert oder ein- oder zweifach durch ($C_1$-$C_3$)-Alkyl, vorzugsweise Methyl, durch ($C_1$-$C_3$)-Alkoxy, vorzugsweise Methoxy, durch Halogen, wie F, Cl, Br, vorzugsweise Chlor, oder durch Methylen substituiert ist und gesättigt ist oder eine oder zwei Doppelbindungen enthalten kann,

R(4) Wasserstoff oder eine übliche Carboxylschutzgruppe, die durch Hydrolyse, Photolyse, Oxidation, Reduktion oder enzymatisch abgespalten werden kann,

R(5) Wasserstoff oder eine übliche Alkoholschutzgruppe, die durch Hydrolyse, Photolyse, Oxidation, Reduktion oder enzymatisch abgespalten werden kann.

Als besonders bevorzugt kommen die folgenden Substituenten in Betracht:

R(1), R(2) Wasserstoff, ($C_1$-$C_4$)-Alkyl, ($C_1$-$C_4$)-Alkenyl, ($C_1$-$C_4$)-Alkoxy, ($C_4$-$C_7$)-Cycloalkyl oder ($C_3$-$C_6$)-Spirocyclyl,

R(3) Wasserstoff, ($C_1$-$C_4$)-Alkyl, (z. B. Methyl, Ethyl, Hydroxymethyl und Aminomethyl), ($C_1$-$C_4$)-Hydroxyalkyl, ($C_1$-$C_3$)-Alkylthio (z. B. Methylthio, Ethylthio und Propylthio, Methoxycarbonylmethylthio), Phenyl (z. B. 4-Carboxamidophenyl, geschütztes 3,4-Dihydroxyphenyl, 4-Fluorphenyl oder 4-Cyanophenyl), Heterocyclyl, z.B. Pyridyl, Phenylthio, gesättigtes oder ungesättigtes ($C_5$-$C_6$)-Oxacycloalkyl (z. B. Tetrahydrofuryl oder Furyl), ($C_4$-$C_6$)-Oxocycloalkyl (z. B. 1-Oxo-cyclobut-3-yl), 3-Hydroxyiminocyclobutyl, 3-Methoxyiminocyclobutyl und 3,3-Dimethoxycyclobutyl,

R(4) eine Carboxylschutzgruppe wie Allyl, p-Nitrobenzyl oder Trimethylsilylethyl,

R(5) Wasserstoff oder eine Alkohol-Schutzgruppe wie Trimethylsilyl, Dimethyl-tert.-butylsilyl, Diphenyl-tert.-butylsilyl, Allyloxycarbonyl, Trichlorethyloxycarbonyl oder 4-Nitrobenzyloxycarbonyl.

Die Asymmetriezentren C-1, C-5, C-6 und C-8 können sowohl in der R- als auch in der S-Konfiguration vorliegen.

Nach dem erfindungsgemäßen Verfahren werden die Verbindungen der Formel I durch Umsetzen einer Verbindung der Formel IV

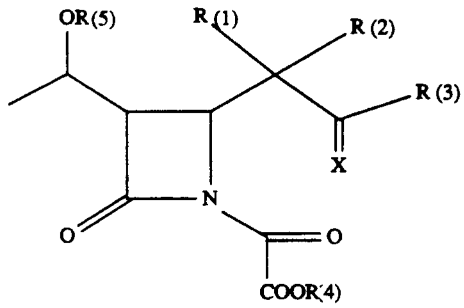

IV

worin X Sauerstoff oder Schwefel bedeutet und R(1), R(2), R(3), R(4) und R(5) die obige Bedeutung haben, mit einer dreiwertigen organischen Phosphorverbindung der Formel V

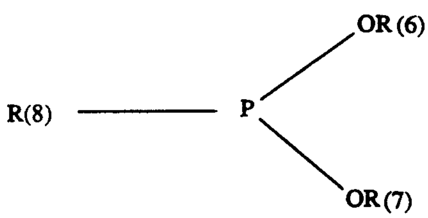

V

hergestellt,
worin bedeuten:

R(6), R(7) ($C_1$-$C_4$)-Alkyl, Allyl, Benzyl oder Phenyl, das substituiert sein kann durch ($C_1$-$C_3$)-Alkyl oder ($C_1$-$C_3$)-Alkoxy, R(6) und R(7) können gleich oder verschieden sein, und

R(8) ($C_1$-$C_4$)-Alkyl, beispielsweise Methyl, Ethyl oder Trifluormethyl, Phenyl, das substituiert sein kann durch ($C_1$-$C_3$)-Alkyl oder ($C_1$-$C_3$)-Alkoxy.

Die Reaktion zwischen einer Verbindung IV und einer Verbindung V kann in einem geeigneten organischen Lösungsmittel durchgeführt werden, beispielsweise in Tetrahydrofuran, Ethylacetat, einem aromatischen Kohlenwasserstoff wie Benzol, Toluol, Xylol oder Mesitylen oder einem halogenierten Kohlenwasserstoff wie Dichlormethan, Trichlormethan oder 1,1,2-Trichlorethan.

Die Reaktionstemperatur kann zwischen 50 und 180 °C, vorzugsweise zwischen 70 und 165 °C variieren.

Die Konzentration der zu cyclisierenden Verbindung IV beträgt zwischen 1 mmol/l und 150 mmol/l, vorzugsweise zwischen 2 mmol/l und 50 mmol/l.

Die Menge der Verbindung V kann zwischen 2 und 8 Moläquivalenten, vorzugsweise zwischen 2 und 6 Moläquivalenten, bezogen auf IV, betragen.

Die als Nebenprodukte gebildeten Alkylphosphonsäuredialkylester und Alkylthiophosphonsäuredialkylester können auf einfache Weise abgetrennt werden.

Die Verbindungen der Formeln IV und V sind bekannt oder können gemäß literaturbekannten Verfahren hergestellt werden.

Die folgenden Beispiele dienen zur weiteren Erläuterung der Erfindung.

Beispiel 1

(1R,5S,6S)-6-[(1R)-tert.-Butyldimethylsilyloxyethyl)-2-(methoxycarbonylmethylthio)-1-methylcarbapen-2-em-3-carbonsäureallylester

Eine Lösung von 1,0 g (2 mmol) (3S,4S)-1-Allyloxycarbonylcarbonyl-3-[(1R)-tert-butyldimethylsilyloxyethyl)-4-[(2R)-1-methoxycarbonylmethylthio-1-oxopropyl]-azetidin-2-on in 50 ml trockenem Xylol wird zum Sieden erhitzt und 1,36 g (10 mmol)
Methylphosphonigsäurediethylester werden zugegeben. Nach 15 Minuten wird abgekühlt, die Lösung im Vakuum eingeengt und der Rückstand über Kieselgel, (desaktiviert mit 10 % Wasser) mit Toluol :

Essigester (10:1) chromatographiert. Nach dem Einengen der Produktfraktionen erhält man einen farblosen, kristallinen Feststoff. Ausbeute: 740 mg (78 % d. Th.)

Analog wurden die in der Tabelle 1 aufgeführten Verbindungen I aus den in Tabelle 2 aufgeführten Ausgangssubstanzen IV unter den in Tabelle 3 aufgeführten Reaktionsbedingungen erhalten.

Tabelle 1 [1]

NMR-Daten der erfindungsgemäßen Peneme I

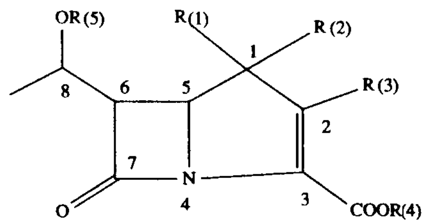

I

| Beispiel | -R(1) | -R(2) | -R(3) | -R(4) | -R(5) | $^1$H-NMR270 MHz ($CDCl_3$): δ [ppm] |
|---|---|---|---|---|---|---|
| 1 | -H | $-CH_3$ | $-SCH_2CO_2CH_3$ | $-CH_2CH=CH_2$ | -TBDMS | 0,10 (s, 6H, $-Si(CH_3)_2$); 0,88 (s, 9H, $SiC(CH_3)_3$; 1,21 (d, J=7 Hz, 3H, -CH-C$\underline{H}_3$); 1,25 (d, J=6 Hz, 3H, -CH-C$\underline{H}_3$); 3,21 (dd, J=3 u. 6Hz 1H, H-6); 3,48 (m, 1H, H-1); 3,42, 3,62 (AB, 2H, $-SCH_2CO_2$-); 3,75 (s, 3H, $-COOCH_3$); 4,16-4,28 (m, 2H, H-5, -C$\underline{H}$-$CH_3$); 4,62-4,83 (m, 2H, $-CO_2CH_2$-); 5,20-5,47 (m, 2H, $=CH_2$); 5,86-6,02 (m, 1H, -CH=). |

[1]TBDMS = tert.-Butyldimethylsilyl, Me = Methyl

Fortsetzung Tabelle 1

| Beispiel | -R(1) | -R(2) | -R(3) | -R(4) | -R(5) | $^1$H-NMR 270 MHz ($CDCl_3$): δ [ppm] |
|---|---|---|---|---|---|---|
| 2 | -H | $-CH_3$ | $-SCH_2CO_2$-p-$C_6H_4$-$NO_2$ | $-CH_2CH{=}CH_2$ | -TBDMS | 0,09-0,10 (2 x s, 3H, $-Si(CH_3)_2$); 0,85 (s, 9H, $-SiC(CH_3)_3$); 1,10-1,30 (2 x d, 6H, $CH-C\underline{H}_3$); 3,20 (dd, J=4 u. 8 Hz, 1H, H-6); 3,40-3,80 (AB u. m, 3H, $-SCH_2CO_2-$ u. H-1); 4,15 (dd, J=4 u. 10 Hz, 1H, H-5); 4,25 (m, 1H, $-C\underline{H}-CH_3$); 4,75 (m, 2H, $-CO_2CH_2-$); 5,20-5,50 (m, 4H, $-CO_2CH_2-$ u. $=CH_2$); 5,85-6,10 (m, 1H, -CH=); 7,50 u. 8,22 (AA'BB', 4H, Aromaten-H). |
| 3 | -H | $-CH_3$ | $-SCH_2CH_2CO_2CH_3$ | $-CH_2CH{=}CH_2$ | -TBDMS | 0,10 (s, 6H, $-Si(CH_3)_2$); 1,25 (d, J=4 Hz, 3H, $-CH-C\underline{H}_3$); 1,28 (d, J = 2 Hz, 3H, $-CH-C\underline{H}_3$); 2,60-2,70, 3,00-3,15 (2 x m, 4H, $-S-C\underline{H}_2-C\underline{H}_2-CO_2-$); 3,20 (dd, J = 3 u. 6 Hz), H-6); 3,25-3,45 (m, 1H, H-1); 3,72 (s, 3H, $-CO_2CH_3$); 4,17 (dd, J = 3 u. 7 Hz, 1H, H-5); 4,19-4,30 (m, 1H, $-C\underline{H}-CH_3$); 4,60-4,88 (m, 2H, $-CO_2CH_2-$); 5,20-5,50 (m, 2H, $=CH_2$); 5,85-6,05 (m, 1H, -CH=). |
| 4 | -H | -H | $-C_6H_5$ | $-CH_2CH{=}CH_2$ | -TBDMS | 0,10 (s, 6H, $-Si(CH_3)_2$); 0,90 (s, 9H, $-SiC(CH_3)_3$); 1,27 (d, J = 8 Hz, 3H, |

Fortsetzung Tabelle 1

| Beispiel | -R(1) | -R(2) | -R(3) | -R(4) | -R(5) | $^1$H-NMR 270 MHz ($CDCl_3$): δ [ppm] |
|---|---|---|---|---|---|---|
| | | | | | | $-CH-C\underline{H}_3$); 3,1-3,35 (m, 3H, H-6 u. H-1); 4,18-4,32 (m, 2H, H5 u. $-C\underline{H}-CH_3$); 4,55-4,78 (m, 2H, $-CO_2CH_2-$); 5,15-5,32 (m, 2H, $=CH_2$); 5,75-5,94 (m, 1H, -CH=); 7,30-7,41 (m, 5H, Aromaten-H). |
| 5 | -H | -H | $-C_6H_5$ | $-CH_2CH_2SiMe_3$ | -TBDMS | 0,01 (s, 9H, $-Si(CH_3)_3$); 0,10 (s, 6H, $-SiC(CH_3)_2$); 0,91 (s, 9H, $-SiC(CH_3)_3$); 0,95-1,03 (m, 2H, $-CH_2-C\underline{H}_2-Si(CH_3)_3$); 1,29 (d, J = 7 Hz, 3H, $CH-C\underline{H}_3$); 3,10-3,37 (m, 3H, H-1 u. H-6); 4,21-4,30 (m, 4H, $-C\underline{H}_2-CH_2-Si(CH_3)_3$; H-5 u. $-C\underline{H}-CH_3$); 7,3-7,4 (m, 5H, Aromaten-H). |
| 6 | -H | $-CH_3$ | $-C_6H_5$ | $-CH_2CH=CH_2$ | -TBDMS | 0,11, 0,12 (2 x s, 6H, $-Si(CH_3)_2$); 0,89 (s, 9H, $-SiC(CH_3)_3$); 1,04 (d, J = 8 Hz, 3H, $-CH-C\underline{H}_3$); 1,28 (d, J = 7 Hz, 3H, $-CH-C\underline{H}_3$); 3,30 (dd, J = 4 u. 8 Hz, 1H, H-6); 3,39 (dq, J = 7 u. 10 Hz, 1H, H-1); 4,22-4,45 (m, 2H, $-C\underline{H}-CH_3$); 4,52-4,70 (m, 2H, $-CO_2CH_2-$); 5,1-5,3 (m, 2H, $=CH_2$); 5,62-5,89 (m, 1H, -CH=); 7,3-7,4 (m, 5H, Aromaten-H). |

Fortsetzung Tabelle 1

| Beispiel | -R(1) | -R(2) | -R(3) | -R(4) | -R(5) | $^1$H-NMR 270 MHz ($CDCl_3$): δ [ppm] |
|---|---|---|---|---|---|---|
| 7 | -H | $-CH_3$ | $-SC_6H_5$ | $-CH_2$-p-$C_6H_4$-$NO_2$ | -TBDMS | 0,04, 0,05 (2 x s, 3H, $-Si(CH_3)_2$); 0,85 (s, 9H, $-SiC(CH_3)_3$); 0,95 (d, J = 8 Hz, 3H, $-CH-C\underline{H}_3$); 1,15 (d, J = 8 Hz, 3H, $-CH-C\underline{H}_3$); 2,95-3,15 (m, 1H, H1); 3,20 (dd, J = 4 u. 8 Hz, H-6); 4,20 (dd, J = 4 u. 12 Hz, H-5); 4,21-4,35 (m, 1H, $-C\underline{H}-CH_3$); 5,30 u. 5,50 (AB, 2H, $-CO_2CH_2-$); 7,30-7,45, 7,50-7,60 (m, 5H, Aromaten-H); 7,69, 8,22 (AA'BB', 4H, Aromaten-H). |
| 8 | $-CH_3$ | $-CH_3$ | $-C_6H_5$ | $-CH_2CH=CH_2$ | -TBDMS | 0,11 (s, 6H, $-Si(CH_3)_2$); 0,91 (s, 9H, $-SiC(CH_3)_3$); 1,16 (s, 6H, $-C(CH_3)_2$); 1,31 (d, J = 7 Hz, 3H, $-CH-C\underline{H}_3$); 3,24 (dd, J = 2 u. 7 Hz, 1H, H-6); 3,89 (d, J = 2 Hz, 1H, H-5), 4,19-4,31 (m, 1H, $-C\underline{H}-CH_3$); 4,44-4,63 (m, 2H, $-CO_2CH_2-$); 5,03-5,15 (m, 2H, $=CH_2$); 5,60-5,78 (m, 1H, -CH=); 7,05-7,12 (m, 2H, Aromaten-H); 7,3-7,4 (m, 3H, Aromaten-H). |
| 9 | $-CH_3$ | $-CH_3$ | $-C_6H_5$ | $-CH_2CH_2SiMe_3$ | -TBDMS | -0,05 (s, 9H, $-Si(CH_3)_3$); 0,11 (s, 6H, $-Si(CH_3)_2$); 0,78-0,87 (m, 2H, $-CH_2C\underline{H}_2-Si(CH_3)_3$); 0,90 (s, 6H, $-C(CH_3)_2$); |

Fortsetzung Tabelle 1

| Beispiel | -R(1) | -R(2) | -R(3) | -R(4) | -R(5) | $^1$H-NMR 270 MHz ($CDCl_3$): δ [ppm] |
|---|---|---|---|---|---|---|
| | | | | | | 1,31 (d, J = 7 Hz, 3H, -CH-C<u>H</u>$_3$), 3,24 (dd, J = 2 u. 7 Hz, 1H, H-6); 3,87 (d, J = 2 Hz, 1H, H-5); 4,05-4,15 (m, 2H; -C<u>H</u>$_2$-$CH_2Si(CH_3)_3$); 4,18-4,30 (m, 1H, -C<u>H</u>-$CH_3$); 7,05-7,13 (m, 2H, Aromaten-H); 7,3-7,4 (m, 3H, Aromaten-H). |
| 10 | $-(CH_2)_5-$ | | $-C_6H_5$ | $-CH_2CH=CH_2$ | -TBDMS | 0,12 (s, 6H, $-Si(CH_3)_2$); 0,92 (s, 9H, $-SiC(CH_3)_2$); 1,38 (d, J = 7 Hz, 3H, -CH-C<u>H</u>$_3$); 1,24-1,78 (m, 10 H, $-(CH_2)_5-$); 3,47 (dd, J = 2 u. 7 Hz, 1H, H-6), 3,97 (d, J = 2 Hz, H-5); 4,3-4,6 (m, 3H, $-CO_2-CH_2-$ u. -C<u>H</u>-$CH_3$); 5,02-5,15 (m, 2H, $=CH_2$); 5,58-5,73 (m, 1H, -CH=); 7,0-7,1 (m, 2H, Aromaten-H); 7,3-7,4 (m, 3H, Aromaten-H). |
| 11 | -H | -H | $-p-C_6H_4F$ | $-CH_2CH=CH_2$ | -TBDMS | 0,10 (s, 6H, $-Si(CH_3)_2$); 0,90 (s, 9H, $-SiC(CH_3)_3$; 1,29 (d, J = 7 Hz, 3H, -CH-C<u>H</u>$_3$); 3,08-3,3 (m, 3H, H-1 u. H-6); 4,20-4,33 (m, 2H, -C<u>H</u>-$CH_3$ u. H-5); 4.58-4.79 (m, 2H, $-CO_2-CH_2-$); 5,17-5,37 (m, 2H, $=CH_2$); |

Fortsetzung Tabelle 1

| Beispiel | -R(1) | -R(2) | -R(3) | -R(4) | -R(5) | $^1$H-NMR 270 MHz ($CDCl_3$): δ [ppm] |
|---|---|---|---|---|---|---|
| | | | | | | 5,80-5,96 (m, 1H, -CH=); 7,0-7,1 (m, 2H, Aromaten-H); 7,35-7,45 (m, 2H, Aromaten-H). |
| 12 | -H | -H | -o-$C_6H_4F$ | -$CH_2CH=CH_2$ | -TBDMS | 0,10 (s, 6H, -Si($CH_3$)$_2$); 0,90 (s, 9H, -SiC($CH_3$)$_3$; 1,29 (d, J = 7 Hz, 3H, -CH-$CH_3$); 3,05-3,37 (m, 3H, H-1 u. H-6); 4,20-4,37 (m, 2H, H-5 u. -CH-$CH_3$); 4,55-4,72 (m, 2H, -$CO_2$-$CH_2$-); 5,10-5,29 (m, 2H, =$CH_2$); 5,73-5,90 (m, 1H, -CH=); 7,0-7,4 (m, 4H, Aromaten-H). |
| 13 | -H | -H | -p-$C_6H_4$-CN | -$CH_2CH=CH_2$ | -TBDMS | 0,10 (s, 6H, -Si($CH_3$)$_2$); 0,90 (s, 9H, -SiC($CH_3$)$_3$); 1,28 (d, J = 7 Hz, 3H, -CH-$CH_3$); 3,10-3,38 (m, 3H, H-6 u. H-1); 4,22-4,38 (m, 2H, H-5 u. -CH-$CH_3$); 4,59-4,78 (m, 2H, -$CO_2$-$CH_2$-); 5,18-5,38 (m, 2H, =$CH_2$); 5,78-5,95 (m, 1H, -CH=); 7,47, 7,62 (AA'BB', 4H, Aromaten-H). |
| 14 | -H | -H | -$CH_2$OTBDMS | -$CH_2$-p-$C_6H_4$-$NO_2$ | -TBDMS | 0,09, 0,10 (2 x s, 2 x 3H, -Si($CH_3$)$_2$); 0,90, 0,95 (2 x s, 2 x 9H, 2 x SiC($CH_3$)$_3$); 1,28 (d, J = 7 Hz, 3H, -CH-$CH_3$); 3,10-3,40 (m, 3H, |

Fortsetzung Tabelle 1

| Beispiel | -R(1) | -R(2) | -R(3) | -R(4) | -R(5) | $^1$H-NMR 270 MHz ($CDCl_3$): δ [ppm] |
|---|---|---|---|---|---|---|
| | | | | | | H-1 u. H-6); 4,10-4,40 (m, 2H, $-C\underline{H}-CH_3$ u. H-5); 4,75 (s, 2H, $-C_6H_4-C\underline{H_2}-$); 5,10-5,42 (AB, 2H, $-C\underline{H_2}-C_6H_4-NO_2$); 7,28-7,40 (AA'BB', 4H, $-C_6\underline{H_4}-CH_2-$); 7,48 u. 8,17 (AA'BB', 4H, $-CH_2-C_6\underline{H_4}-NO_2$). |
| 15 | -H | -H | 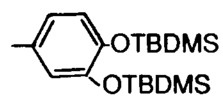 | $-CH_2CH=CH_2$ | -TBDMS | 0,10 (s, 6H, $-Si(CH_3)_2$); 0,20, 0,21 (2 x s, 6H, $-Si(CH_3)_2$); 0,89 (s, 9H, $-SiC(CH_3)_3$); 0,98 (s, 18 H, $-SiC(CH_3)_3$); 1,27 (d, J = 7 Hz, 3H, $-CH-C\underline{H_3}$); 3,05-3,30 (m, 3H, H-1 u. H-6); 4,17-4,31 (m, 2H, $-C\underline{H}-CH_3$ u. H-5); 4,60-4,78 (m, 2H, $-CO_2-CH_2-$), 5,15-5,36 (m, 2H, $=CH_2$); 5,80-5,96 (m, 1H, CH=); 6,75-6,95 (m, 3H, Aromaten-H). |

Tabelle 2 [2]
Ausgangsverbindungen

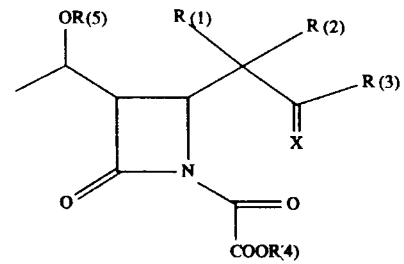

IV

| Beispiel | -R(1) | -R(2) | -R(3) | -R(4) | -R(5) | =X |
|---|---|---|---|---|---|---|
| 1 | -H | $-CH_3$ | $-SCH_2CO_2CH_3$ | $-CH_2CH=CH_2$ | -TBDMS | =O |
| 2 | -H | $-CH_3$ | $-SCH_2CO_2$-p-$C_6H_4$-$NO_2$ | $-CH_2CH=CH_2$ | -TBDMS | =O |
| 3 | -H | $-CH_3$ | $-SCH_2CH_2CO_2CH_3$ | $-CH_2CH=CH_2$ | -TBDMS | =O |
| 4 | -H | -H | $-C_6H_5$ | $-CH_2CH=CH_2$ | -TBDMS | =O |
| 5 | -H | -H | $-C_6H_5$ | $-CH_2CH_2-SiMe_3$ | -TBDMS | =O |
| 6 | -H | $-CH_3$ | $-C_6H_5$ | $-CH_2CH=CH_2$ | -TBDMS | =O |
| 7 | -H | $-CH_3$ | $-SC_6H_5$ | $-CH_2$-p-$C_6H_4$-$NO_2$ | -TBDMS | =O |
| 8 | $-CH_3$ | $-CH_3$ | $-C_6H_5$ | $-CH_2CH=CH_2$ | -TBDMS | =O |
| 9 | $-CH_3$ | $-CH_3$ | $-C_6H_5$ | $-CH_2CH_2SiMe_3$ | -TBDMS | =O |
| 10 | $-(CH_2)_5-$ | | $-C_6H_5$ | $-CH_2CH=CH_2$ | -TBDMS | =O |
| 11 | -H | -H | -p-$C_6H_4$-F | $-CH_2CH=CH_2$ | -TBDMS | =O |
| 12 | -H | -H | -o-$C_6H_4$-F | $-CH_2CH=CH_2$ | -TBDMS | =O |
| 13 | -H | -H | -p-$C_6H_4$-CN | $-CH_2CH=CH_2$ | -TBDMS | =O |
| 14 | -H | -H | 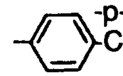 $-CH_2OTBDMS$ | $-CH_2CH=CH_2$ | -TBDMS | =O |
| 15 | -H | -H | 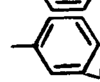 -OTBDMS, OTBDMS | $-CH_2CH=CH_2$ | -TBDMS | =O |

[2] TBDMS = tert.-Butyldimethylsilyl, Me = Methyl

Tabelle 3
Reaktionsbedingungen für die Zyklisierungsreaktion

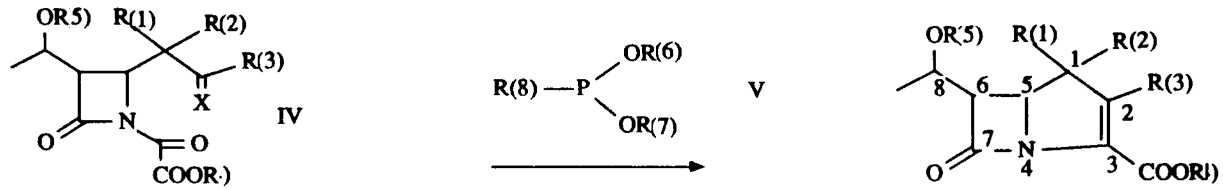

IV V I

| Beispiel | Reagenz V | Äquivalente V | Konz. (mmol/l) | Lösungs-mittel | Temp. (°C) | Reaktions-zeit (h) | Ausbeute (%) |
|---|---|---|---|---|---|---|---|
| 1 | $CH_3P(OC_2H_5)_2$ | 5 | 40 | Xylol | 140°C | 0,25 | 78 |
| 2 | $CH_3P(OC_2H_5)_2$ | 5 | 33 | Xylol | 140°C | 0,25 | 44 |
| 3 | $CH_3P(OC_2H_5)_2$ | 5 | 40 | Xylol | 140°C | 0,50 | 69 |
| 4 | $CH_3P(OC_2H_5)_2$ | 3 | 110 | m-Xylol | 140°C | 0,25 | 53 |
| 5 | $CH_3P(OCH_3)_2$ | 8 | 100 | m-Xylol | 130°C | 0,33 | 41 |
| 6 | $CH_3P(OC_2H_5)_2$ | 3 | 67 | m-Xylol | 140°C | 6,00 | 53 |
| 7 | $CH_3P(OC_2H_5)_2$ | 5 | 40 | Xylol | 140°C | 0,25 | 72 |
| 8 | $CH_3P(OC_2H_5)_2$ | 5 | 33 | m-Xylol | 135°C | 0,75 | 67 |
| 9 | $CH_3P(OCH_3)_2$ | 8 | 100 | Toluol | 110°C | 24,00 | 39 |
| 10 | $CH_3P(OC_2H_5)_2$ | 5 | 33 | m-Xylol | 140°C | 0,50 | 79 |
| 11 | $CH_3P(OC_2H_5)_2$ | 3 | 110 | m-Xylol | 140°C | 0,25 | 56 |
| 12 | $CH_3P(OC_2H_5)_2$ | 3 | 115 | m-Xylol | 140°C | 0,25 | 55 |
| 13 | $CH_3P(OC_2H_5)_2$ | 3 | 100 | m-Xylol | 140°C | 0,25 | 36 |
| 14 | $CH_3P(OC_2H_5)_2$ | 4 | 32 | Xylol | 140°C | 0,33 | 49 |
| 15 | $CH_3P(OC_2H_5)_2$ | 3 | 50 | m-Xylol | 140°C | 2,00 | 54 |

## Patentansprüche

**1.** Verfahren zum Herstellen einer Carbapenem-Verbindung I

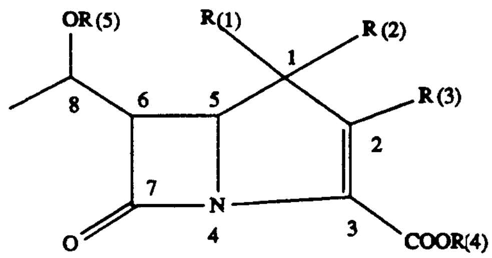

I

worin bedeuten:

R(1), R(2) Wasserstoff, $(C_1-C_4)$-Alkyl, $(C_1-C_4)$-Alkenyl, $(C_1-C_4)$-Alkoxy, $(C_4-C_7)$-Cycloalkyl oder $(C_3-C_6)$-Spirocyclyl,

R(3) Wasserstoff, $(C_1-C_4)$-Alkyl, $(C_1-C_{12})$-Alkylthio [wobei die Alkylgruppen unsubstituiert sind oder ein- oder zweifach substituiert durch Hydroxy, geschütztes Hydroxy, $(C_1-C_4)$-Alkoxy, $(C_1-C_4)$-Alkyloxycarbonyl,$(C_1-C_4)$-Acyloxy, Amino, $(C_1-C_4)$-Alkylamino, $(C_1-C_4)$-Acylamino, Thiol, $(C_1-C_4)$-Alkylthio oder Heterocyclylthio], Phenyl, Heterocyclyl, Phenylthio, Heterocyclylthio [wobei die Phenyl- und Heterocyclylringe unsubstituiert oder ein- oder zweifach substituiert sind durch Hydroxy, geschütztes Hydroxy, Carboxy, $(C_1-C_4)$-Alkoxycarbonyl, Allyloxycarbonyl, Aminocarbonyl, $(C_1-C_4)$-Alkylaminocarbonyl, Cyano, F, Cl, Br], $(C_3-C_6)$-Cycloalkyl, $(C_3-C_6)$-Cycloalkylthio, $(C_5-C_6)$-Oxacycloalkyl [gesättigt, ein- oder zweifach ungesättigt], $(C_3-C_6)$-Oxocycloalkyl, $(C_3-C_6)$-[1,1-bis-$(C_1-C_3)$-Alkyloxy]-cycloalkyl, $(C_3-C_6)$-$(C_1-C_3)$-Alkylimino]-cycloalkyl, $(C_3-C_6)$-(Phenylimino)-cycloalkyl, $(C_3-C_6)$-(Hydroxyimino)-cycloalkyl, $(C_3-C_6)$-[$(C_1-C_3)$-Alkyloxyimino]-cycloalkyl, in denen der Cycloalkylrest unsubstituiert oder ein- oder zweifach durch $(C_1-C_3)$-Alkyl, durch $(C_1-C_3)$-Alkoxy, durch Halogen, oder durch Methylen substituiert ist und gesättigt ist oder eine oder zwei Doppelbindungen enthalten kann,

R(4) Wasserstoff oder eine übliche Carboxylschutzgruppe, die durch Hydrolyse, Photolyse, Oxidation, Reduktion oder enzymatisch abgespalten werden kann,

R(5) Wasserstoff oder eine Alkohol-Schutzgruppe,

dadurch gekennzeichnet, daß man eine Verbindung der Formel IV

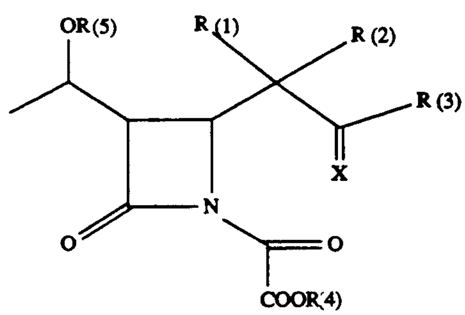

IV

worin

X Sauerstoff oder Schwefel bedeutet und

R(1), R(2), R(3), R(4) und R(5) die obige Bedeutung haben, mit einer dreiwertigen organischen Phosphorverbindung der Formel V

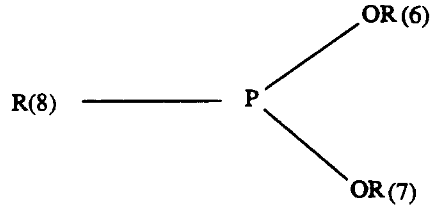

V

umsetzt,
worin

R(6), R(7) ($C_1$-$C_4$)-Alkyl, Allyl, Benzyl oder Phenyl, das substituiert sein kann durch ($C_1$-$C_3$)-Alkyl oder ($C_1$-$C_3$)-Alkoxy, R(6) und R(7) können gleich oder verschieden sein, und

R(8) ($C_1$-$C_4$)-Alkyl, Phenyl, das substituiert sein kann durch ($C_1$-$C_3$)-Alkyl oder ($C_1$-$C_3$)-Alkoxy, bedeuten.

**2.** Verfahren nach Anspruch 1, dadurch gekennzeichnet, daß man die Umsetzung von Verbindung IV mit V in einem organischen Lösungsmittel durchführt.

**3.** Verfahren nach Anspruch 1, dadurch gekennzeichnet, daß man die Reaktion zwischen +50 °C und +180 °C durchführt.

**4.** Verfahren nach Anspruch 1, dadurch gekennzeichnet, daß

R(1), R(2) Wasserstoff, ($C_1$-$C_4$)-Alkyl, ($C_1$-$C_4$)-Alkenyl, ($C_1$-$C_4$)-Alkoxy, ($C_4$-$C_7$-)Cycloalkyl oder ($C_3$-$C_6$)-Spirocyclyl,

R(3) Wasserstoff, ($C_1$-$C_4$)-Alkyl, Hydroxy-($C_1$-$C_4$)-Alkyl, ($C_1$-$C_3$)-Alkylthio, Phenyl, Heterocyclyl, Phenylthio, gesättigtes oder ungesättigtes ($C_5$-$C_6$)-Oxacycloalkyl, ($C_4$-$C_6$)- Oxocycloalkyl, 3-Hydroxyiminocyclobutyl, 3-Methoxyiminocyclobutyl und 3,3-Dimethoxycyclobutyl,

R(4) eine Carboxylschutzgruppe ausgewählt aus der Gruppe Allyl, p-Nitrobenzyl oder Trimethylsilylethyl,

R(5) Wasserstoff oder eine Alkohol-Schutzgruppe ausgewählt aus der Gruppe Trimethylsilyl, Dimethyl-tert.bytylsilyl, Diphenyl-tert.-butylsilyl, Allyloxycarbonyl, Trichlorethoxycarbonyl oder 4-Nitrobenzyloxycarbonyl) bedeuten.